# EUROPEAN PATENT APPLICATION

(11) **EP 1 806 100 A1**
(43) Date of publication of application: **11.07.2007**
(21) Application number: 06127326.4
(22) Date of filing: 29.12.2006
(51) Int. Cl.: A61B 10/02

(54) **Sample collector for biopsy and similar**

(30) Priority: 03.01.2006 BR MU8600004 U
(71) Applicant: Machado de Andrade, Eugenio, 04521-002 São Paulo / SP (BR)
(72) Inventor: Machado de Andrade, Eugenio, 04521-002 São Paulo / SP (BR)
(74) Representative: Petraz, Gilberto Luigi

(57) **Abstract**

Sample collector for biopsy and similar, comprising a grip (1), a needle (3) fixed to the grip (1), a disposable stem (4) for collecting tissue sample, and an actuating mechanism (7) located inside said grip. The actuating mechanism comprises one or more movable bases (8A,8B) having on their upper faces a number of projections (10) and on their corners holes (12) inside which are located magnets, and interchangeable intermediate connectors (9A,9B), which have through holes (34) matching said projections (10) for coupling with said bases (8A,8B) by means of said magnets, and on their outer faces fitting elements (2A,2B) for coupling with a standardized set of stems and needles. The sample collector is adaptable to different types of needles (models, brands, sizes) by providing intermediate connectors with fiitting elements having geometries adapted to different needles.

## Description

### FIELD OF APPLICATION

The present invention has as an object a practical and innovative sample collector for biopsy, belonging to the medical field, more specifically to diagnostic medicine, to which was given original constructive disposition, aiming at improving its usage and performance in relation to other models usually found in the market.

### STATE OF THE ART

Diagnostic medicine is an area of medical sciences which has vital importance in a large number of different pathologies. Among these, we can mention one which is of the highest concern to physicians and patients: neoplasies, known as cancer.

The characteristic of cancer is the disarranged multiplication of cells, which lose their growth control and no longer respect the tissue structures.

Later, when cells get into the blood circulation, through vessels and arteries and mainly into the lymphatic circulation, cancer is spread out to other organs. This phase of the disease is known as metastasis, and leads to the organs' failure which may cause death.

The diagnosis of this kind of pathology has always been a challenge to medicine. With the appearance of computerized tomography and image exams, the advance in treatment was remarkable. However, there is a diagnosis resource even more indispensable than those exams: the biopsies.

Biopsy is a clinical exam modality, which consists of the removal and pathological evaluation of tissue samples, such as small fragments of live body. They are extremely important so that the physician can establish a diagnosis and observe the treatment evolution. They are used not only for diagnosis of neoplasies, but also in monitoring cases of transplanted organs, suspicion of insufficiency of organs and tissues, following up of medicine treatments, among others.

Currently, there are two kinds of biopsy: the cytological and the histological one. The cytological biopsy is an aspirating puncture carried out through the use of a thin-sized needle. It is a positive procedure usually guided by ultra-sonography, to aspire cysts or the content of a node or tumor. The histological biopsy consists of a percutaneous procedure, in which through a system of coaxial needles a small sample of tissue is collected where the injury is located. The advantage of histological biopsy over the cytological one is that, by obtaining a sample of removed material instead of aspirated one, groups of complete cells are obtained instead of isolated cells. In addition to that, the collected material is highly concentrated and not diluted by fluids of tissues and blood, as in aspirated biopsy. That eases the microscope examination, rendering it more reliable and suitable to that kind of biopsy.

The histological biopsy is part of a device to protect the needles and aid its handling, the grips, which are constituted of a body which serves as a guide to slide the stem and the collecting needle. The needles and the stems used in the procedure are disposable and intended for a single use, in order to assure the proof efficiency, sterility and uncontestable safety against any kind of accidental inoculation. The instrument grip can be of two main types: the disposable and non-disposable ones.

Among the disposable models are the needles by semi-automatic guillotine-cutter, which are simpler instruments in their construction since they comprise a grip in which the needle itself is already coupled. The model commonly used is of disposable grips, which follow the same principle of needles by semi-automatic guillotine-cutter.

The non-disposable models are similar to the grips of disposable ones; however, they are produced from resistant and sterilizable material, which allows the grip to be repeatedly used after sterilization by heating or by gas.

The state of the art has many shortcomings.

As everybody knows, accuracy and quickness is required from medical people, especially for the surgical area. In order to help to comply with these requirements, there is the advent of surgical materials, such as the grips for collection of biopsy samples previously mentioned. However the current equipment is deficient, and ends up by hindering the surgeon's work even more when carrying out the biopsy.

The disposable models are practical and are more cost-effective than other equipment. However, they do not provide the surgeon with the accuracy necessary for the procedure, being more used in case of injury to tissues closer to tegumentary tissue.

The non-disposable grips are more reliable owing to the needle's coaxial sliding system. However, it is a more expensive equipment, in spite of being reused after sterilization. There is a great number of different needles in the market, with different sizes and lengths, adapted to the organ from where the sample will be collected. There is also a diversity of trademarks and manufacturers of these needles and grips. The main problem with these models is the fact that the grips are not adaptable to the different types of needles, and the physician needs to have a compatible model to each different trademark, size and length.

In addition to this disadvantage, there is also a deficiency in the drive system of the control knob. Owing to the complexity of the system, it becomes difficult for the physician. As those qualified in the surgical area are aware, the collection of biopsy sample is a procedure which requires much attention and accuracy from the surgeon, since the areas to be explored may be millimetrical. The least deviation and the diagnosis is compromised. In the current systems, the triggering drive system and positioning, which takes place through a spring system, is difficult and leads to a higher risk of failures when collecting the tissue.

### DISCLOSURE OF THE INVENTION

It is, therefore, one aspect of the present invention to supply a sample collector for biopsy, with a device in which not only the operational quantities have been considered in the manufacturing design, but also the form, the disposition and the localization of its parts and components which, when correctly positioned, will provide more safety to the process without any cost.

It is therefore a model developed with perfection and efficiency, with the aim at offering a biopsy sample collector, providing more reliability in its purpose, by both its operational characteristics as well the product's easy use.

The device, object of the present invention, contains a discharging mechanism located on the front part of its body. This positioning allows the surgeon to activate the device with his index finger or thumb, providing an accurate placement of the needle to the tissue target place. Since it can be operated with one of the hands, it helps the use in procedures requiring other view instruments, such as ultra-sound devices.

The triggering and positioning of the needle through the grip is delicate and of high risk, since any least deviation may impair the procedure. With that in mind, a safety lock system has been developed which avoids accidental discharge and provides the surgeon with more comfort and reliability when triggering and positioning the needle at the position necessary for collection.

The needle sliding system enables the sample removal without the need of moving the instrument needle, which reduces even further the risk of sample violation, which could contaminate it, thus compromising the result of the examination. The instrument dimensions have been optimized to lessen the trauma risks and maximize the stability during the procedure. This second factor is of paramount importance, in order to provide accuracy of the sample collection, since in this way unnecessary insertion of the stem in several directions can be avoided.

The head fitting system does not require the use of screws or locks, since they are kept positioned through cylindrical fittings and powerful magnets. The lid is able to be articulated and is provided with a magnetic locking system. This differential may be minimal, but it is extremely important when we talk about an instrument used in a surgical procedure, as the case of biopsies. The use of pins, locks or screws require holes to be made on the part. These holes can be the entrance door to bacteria and micro-organisms, which may contaminate the sample, compromising the exam result, or even contaminate the patient himself/herself, causing irreversible damages to his/her health.

The head replacement system allows the use of several different models, brands, sizes and diameters of needles existing in the market, without being necessary to replace the whole part. Just replace the interchangeable elements positioned on the upper faces of the movable bases: instead of a grip for each type of needle, there is a small part, fittable into the grip for each type of needle. Since it is a grip whose parts can be changed so as to be adapted to each type of needle, a constant movement of pins, locks and screws could lead to excessive wear and tear to the equipment. This is an additional advantage of the fixing system by magnets to the consumer.

The object of the present patent is manufactured from sterilizable material, that is, after each sample collection procedure, a sterilization process is carried out through conventional methods. That allows the grip to be re-used in another patient, efficiently and without risks of contamination, in addition to lessening costs with equipment. In case another type of needle with fitting different from the ones existing in the market is developed, new heads with suitable fitting system suitable for each type, showing to be an innovative and highly adaptable device to the market needs, may be developed.

### BRIEF DESCRIPTION OF THE DRAWINGS

These and other characteristics of the present invention will become apparent from the following description of a preferential form of embodiment, given as a non-restrictive example with reference to the attached drawings wherein:

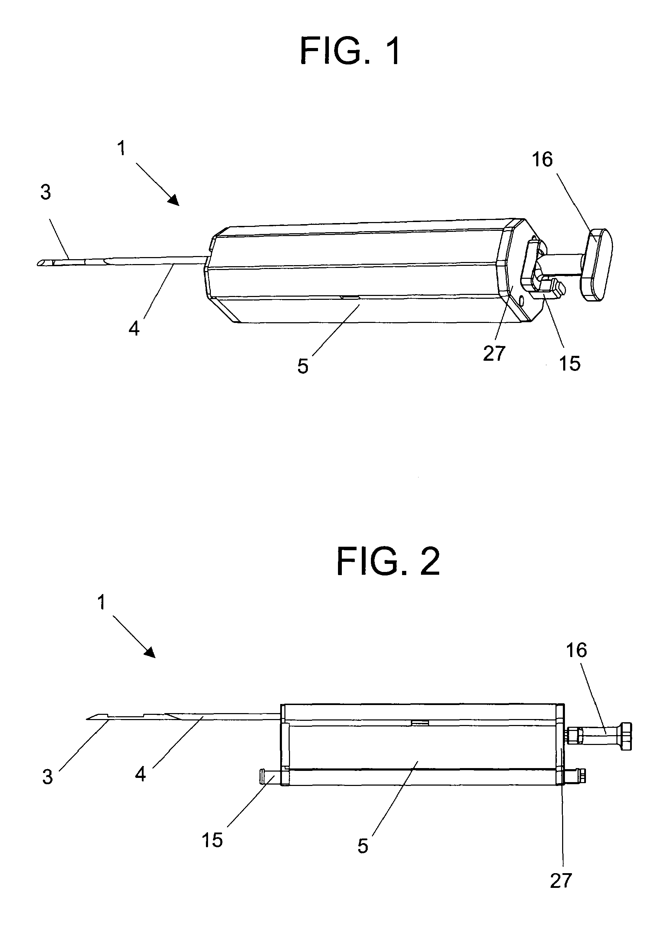

- - fig. 1: shows an upper-back perspective view of the sample collector according to the invention with the grip assembled with needle and stem;
- - fig. 2: shows a side view of the sample collector of figure 1;

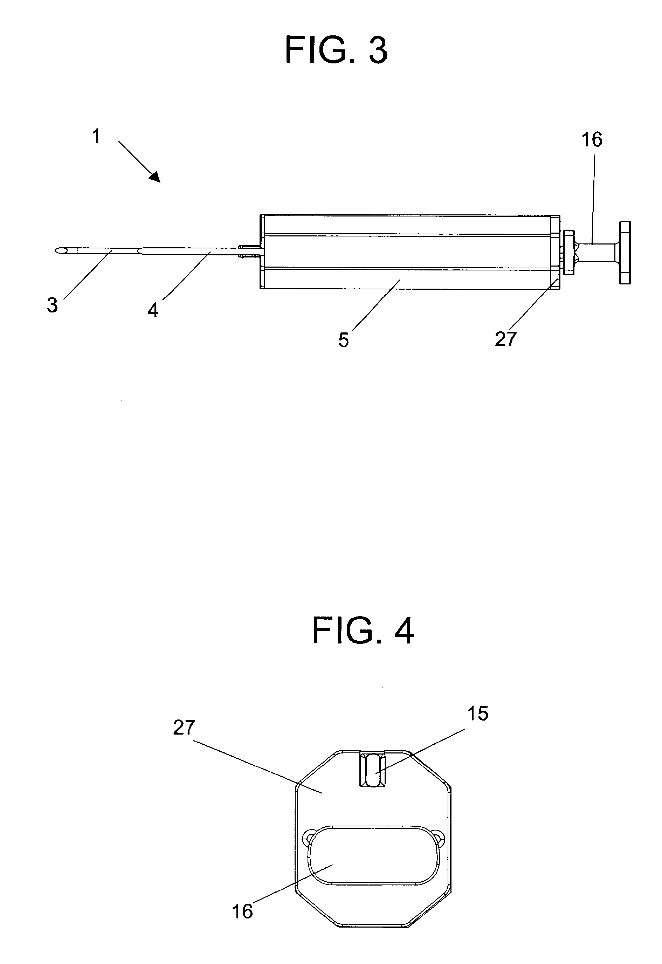

- - fig. 3: shows an upper view of the sample collector of figure 1;
- - fig. 4: shows a back view of the grip, pointing out the handle area;

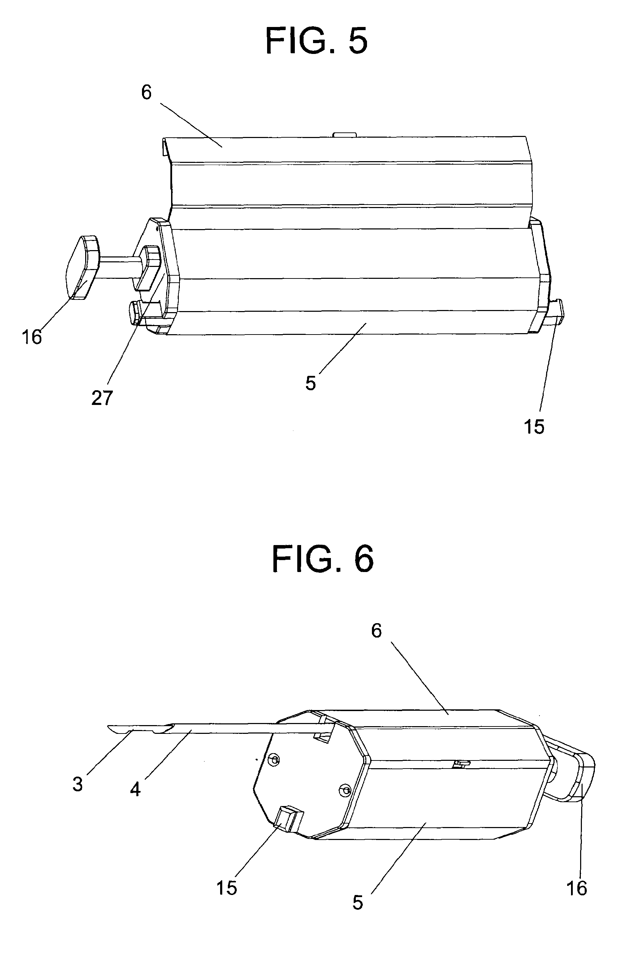

- - fig. 5: shows a side perspective view of the grip with the lid open, without the needle and stem;
- - fig. 6: shows a frontal perspective view of the grip assembled with the needle and stem;

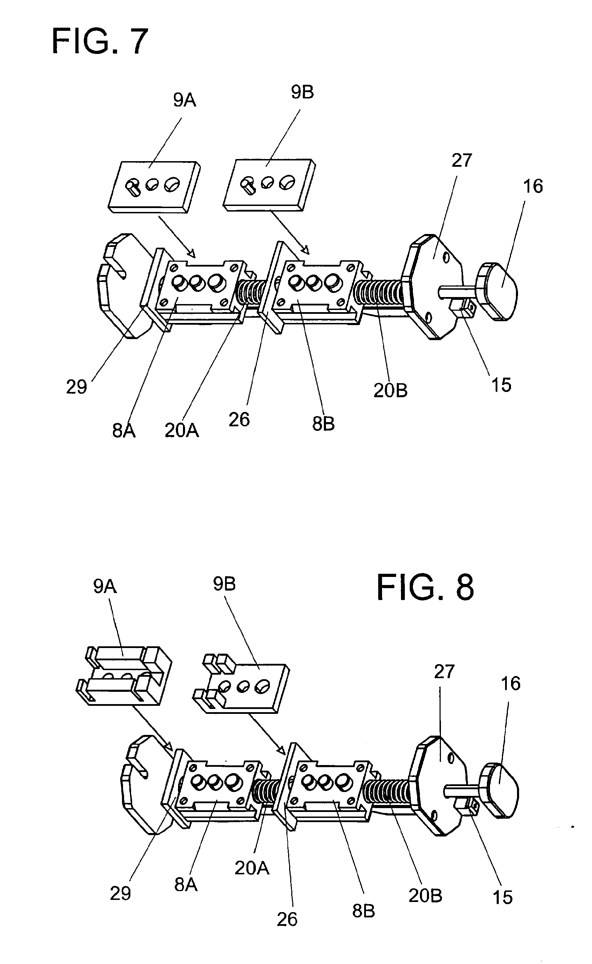

- - fig. 7: shows an upper front perspective view of the needle moving system and of the stem, showing the fitting of the interchangeable elements of the grip;
- - fig. 8: shows a frontal upper perspective view of the needle and stem moving system, showing the fitting of the interchangeable movements, with the needle and stem different from the ones presented in figure 7;
- - fig. 9: shows a side upper perspective view of the moving system of the needle and stem, showing the fitting of the interchangeable movements and of the needle and stem on the grip;
- - fig. 10: shows a side upper perspective view of the moving system of the needle and stem, showing the fitting of the needle and stem on the grip;
- - fig. 11: shows a side upper perspective view of the moving system of the needle and stem, showing the fitting of the interchangeable movements and of the needle and stem on the grip;

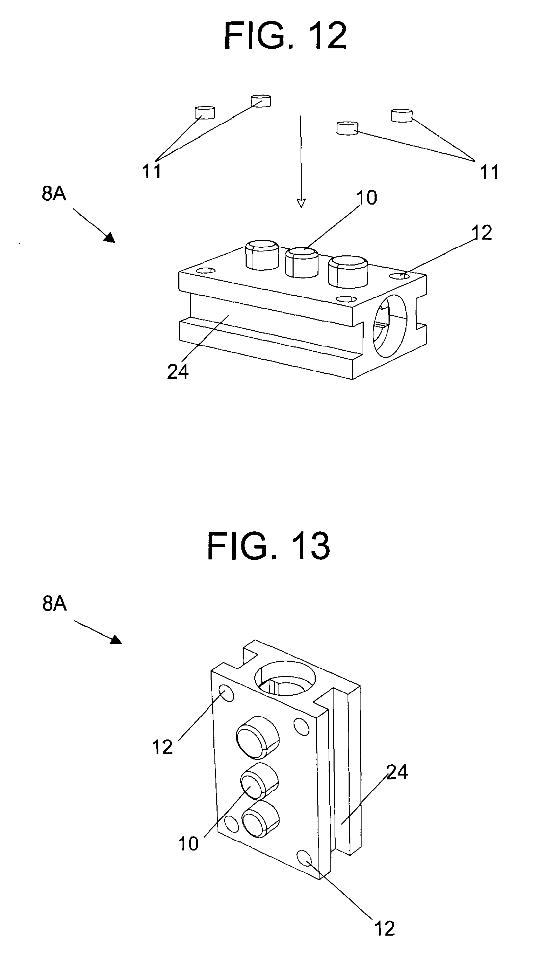

- - fig. 12: shows a side upper perspective view of the base, showing the position of magnet insertion;
- - fig. 13: shows a front upper perspective view of the base;

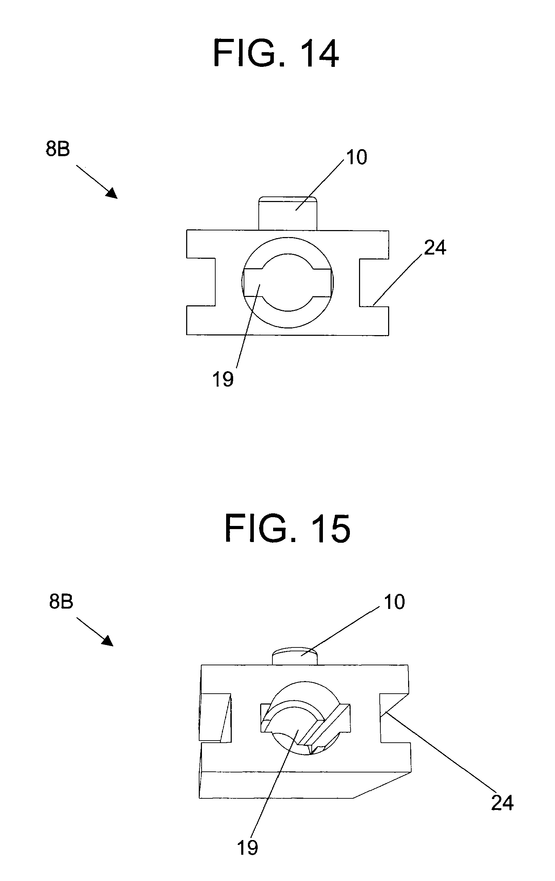

- - fig. 14: shows a frontal view of the base;
- - fig. 15: shows a frontal lower perspective view of the base;

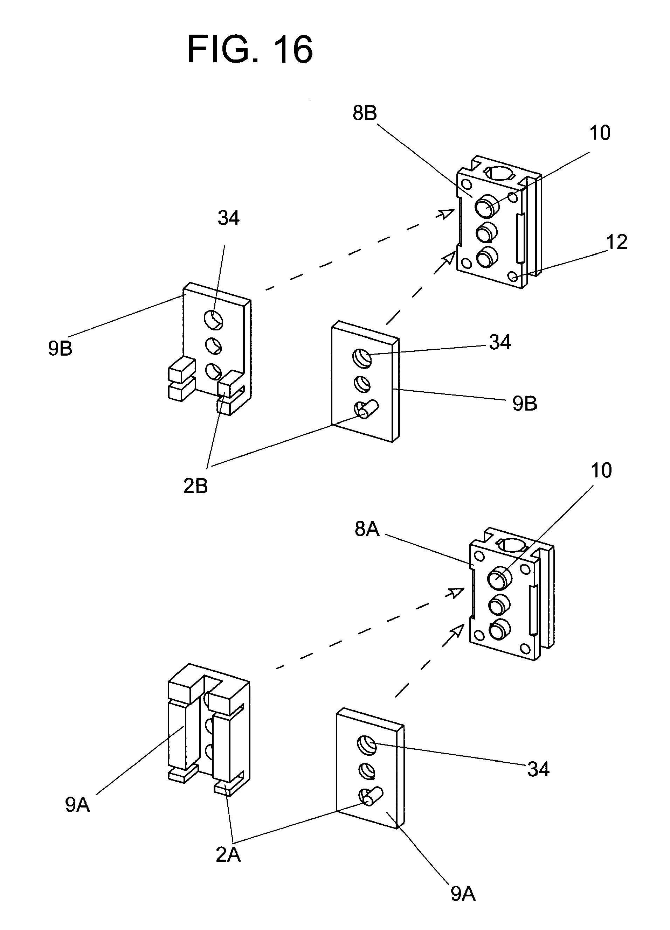

- - fig. 16: shows a frontal upper perspective view of the base of the interchangeable movements, showing the fitting.

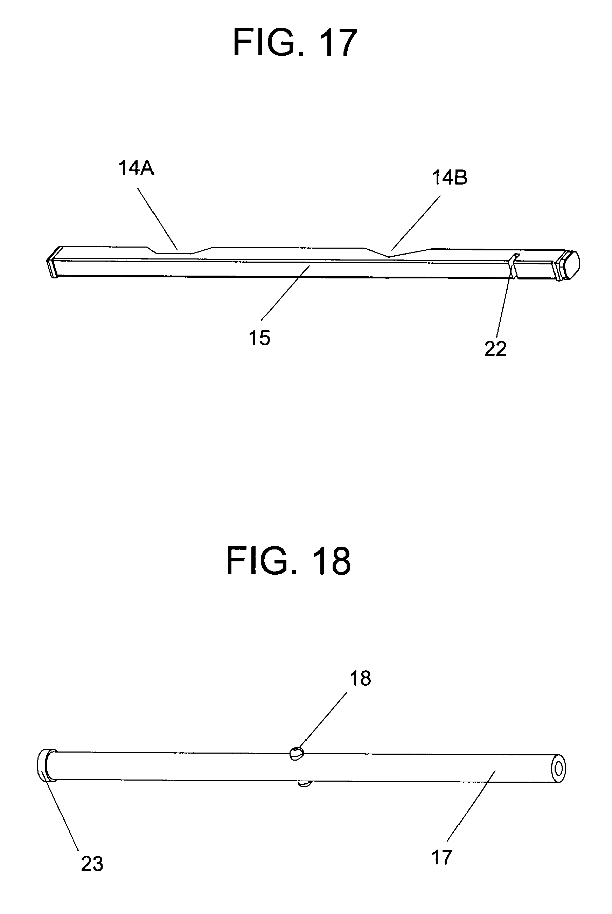

- - fig. 17: shows the side upper perspective view of the rectangular shaft, pointing out the grooves where the safety lock is fitted and the retention sliding elements;
- - fig. 18: shows the side upper perspective view of the cylindrical shaft, pointing out the projections on its central part and its slightly bigger diameter on one of its ends;

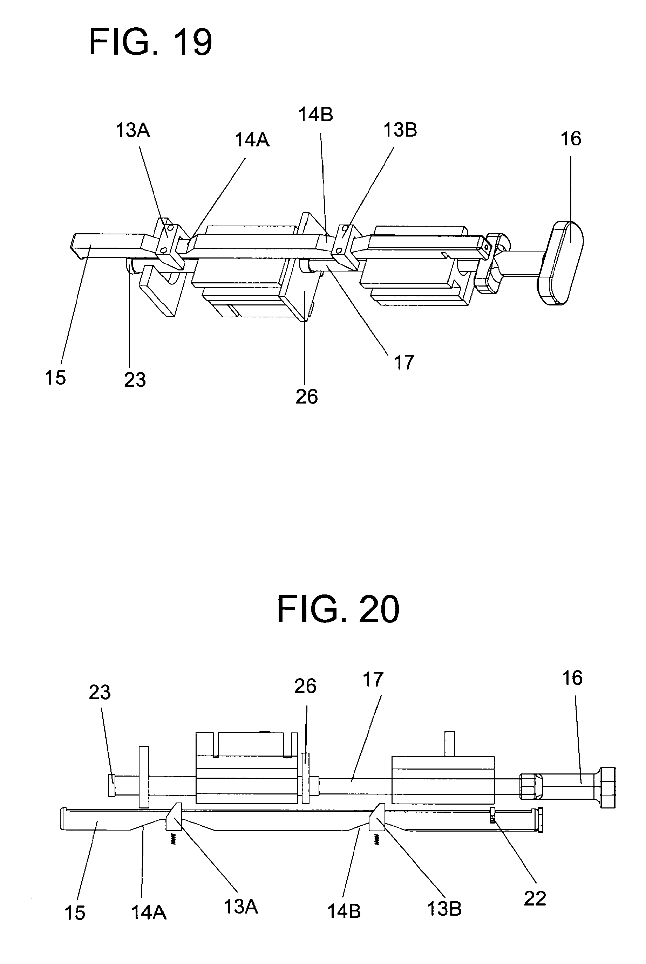

- - fig. 19: shows a side lower perspective view of the system of positioning and locking of the needle and stem;
- - fig. 20: shows a side view of the positioning system and of the locking of needle and stem;

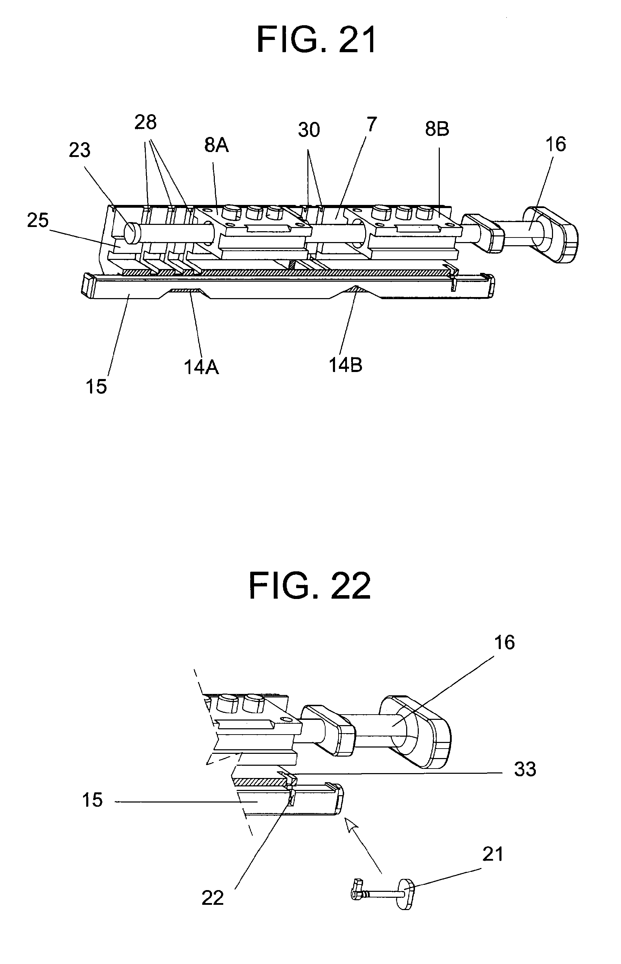

- - fig. 21: shows a side upper perspective view of the grip, with longitudinal cut pointing out the positioning of the rectangular shaft cut in relation to the bases;
- - fig. 22: shows a side upper perspective view of the safety lock system detail of the grip;

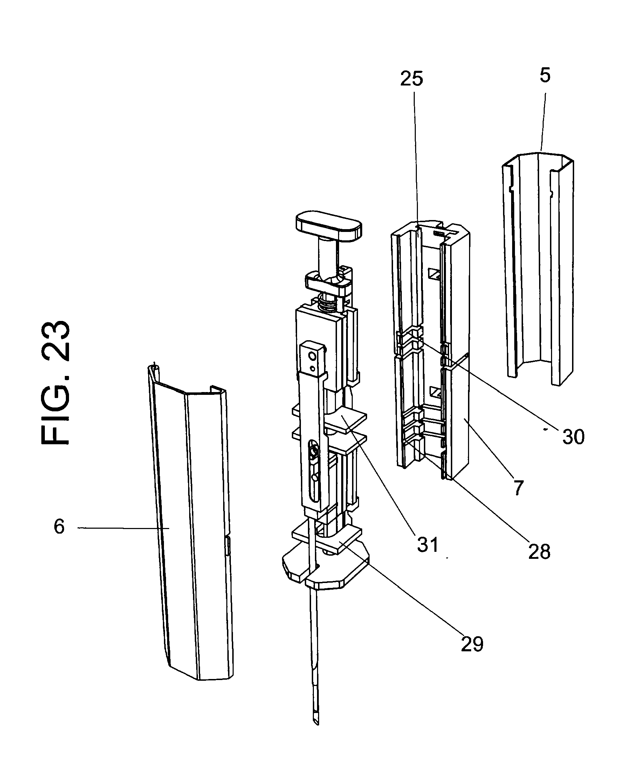

- - fig. 23: shows a blown up side upper perspective view of the grip;

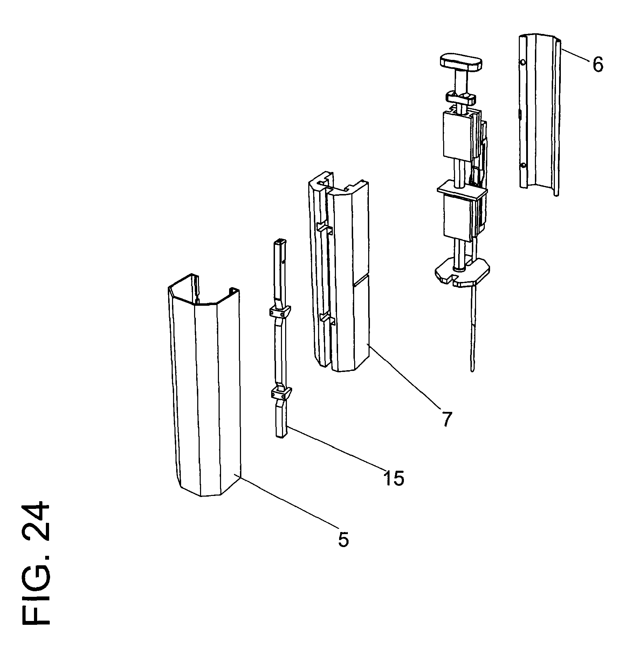

- - fig. 24: shows a blown up side lower perspective view of the grip;

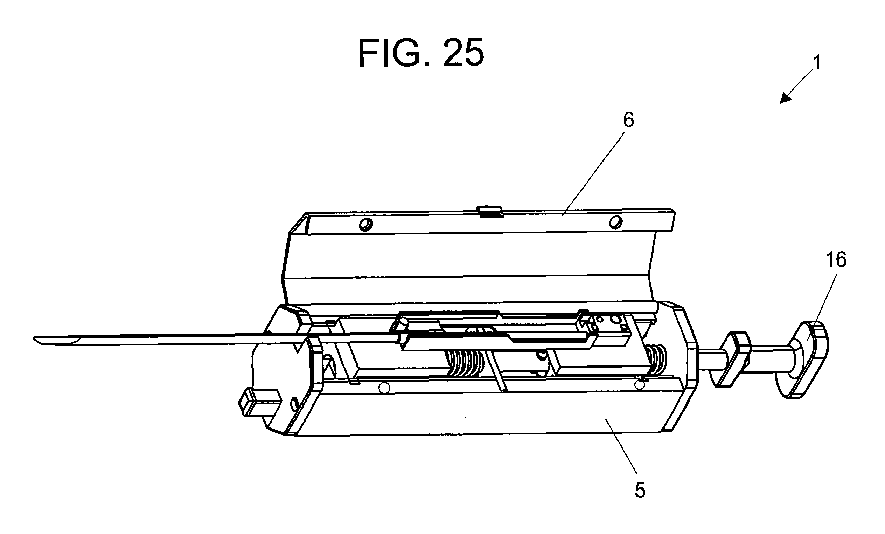

- - fig. 25: shows a frontal upper perspective view of the grip assembled with a certain type of needle and stem;

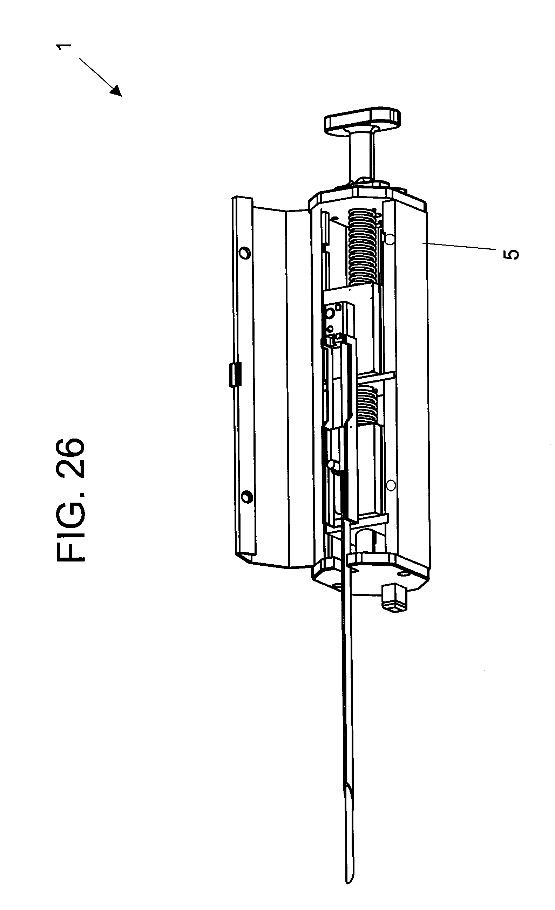

- - fig. 26: shows a frontal upper perspective view of the grip assembled with a type of needle and stem, different from the one presented in figure 25.

### DETAILED DESCRIPTION OF THE PREFERRED EMBODIMENTS

The object of the present invention comprises an improvement for an instrument for biopsy sample collection, which is constituted of a grip 1 which serves as base to a needle 3 and to a disposable stem 4 for collecting tissue sample.

The grip 1 is produced from hard and sterilizable material and it is constituted of a quadrangular structure 5, with an articulated lid 6, which has powerful magnets on its ends for an efficient locking. Inside the grip 1 is located a mono-block actuating mechanism 7, where the components of the grip mechanism are provided.

The referred sample collector has movement which takes place through a system composed of two movable bases 8A and 8B with a parallelepiped shape and having side guides formed by longitudinal cuts 24 of rectangular section on both sides, and fitted into rectangular longitudinal projections 25 provided on the sides of the mono-block mechanism 7. On the upper faces of the bases 8A and 8B are provided, in the embodiment disclosed, three cylindrical projections 10 positioned linearly and longitudinally with respect to the bases 8A and 8B and having sizes progressively larger.

This constructive disposition was idealized aiming at reducing the high costs with the acquisition of equipment. As mentioned before, the current sample collectors of better instrumental quality require the surgeon to have a grip for each type or brand of needle 3 existing in the market.

On the corners of the bases 8A and 8B are provided holes 12 which have powerful magnets 11 located within them. On their upper face are fitted interchangeable elements called intermediate connectors 9A and 9B, which have through holes 34 matching the cylindrical projections 10. The upper face of the intermediate connectors 9A and 9B has fitting elements 2A and 2B standardized with the set of stems 4 and needles 3 existing in the market, that is, the surgeon needs only to acquire a single grip 1 and only the intermediate connectors 9A and 9B for adapting the instrument to the use of a new type of needle 3 necessary to carry out the procedure. That is, the interchangeable elements 9A and 9B have a fitting position defined by the holes 34 provided on their body and by the cylindrical projections 10 of the bases 8A and 8B, and they are fixed by magnets 11 fitted on the bases 8A and 8B.

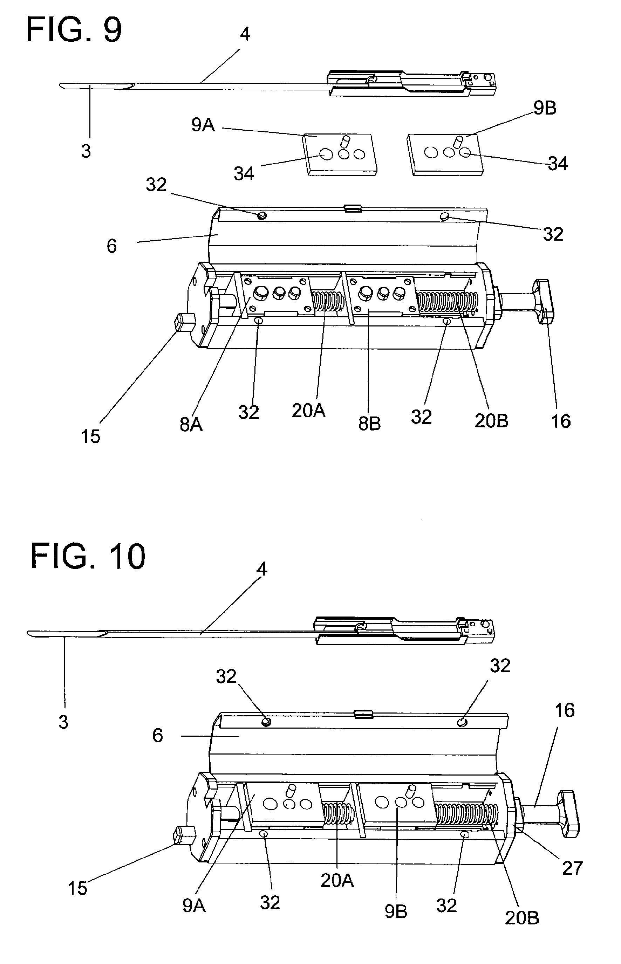

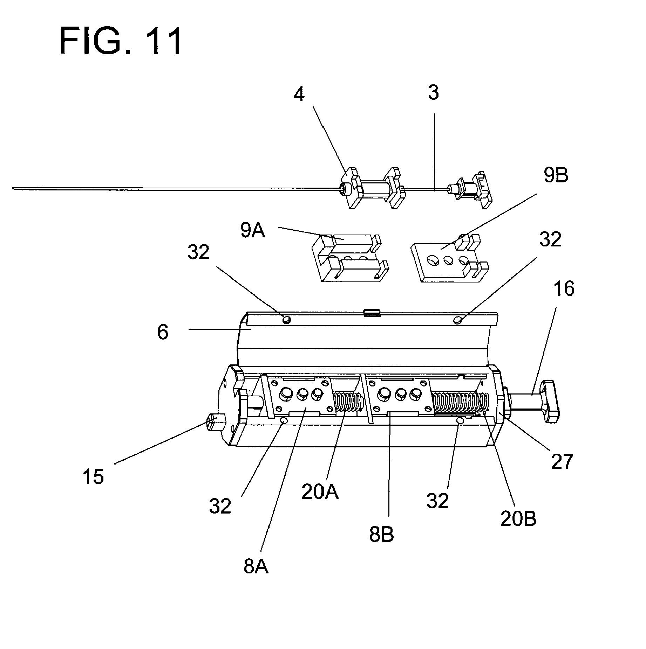

The referred grip 1 was designed to enable the coupling of several types of needle and stem sets existing in the market, through the use of intermediate connectors 9A and 9B adapted to the set of needle and stem, which is intended to be used. Figure 9 and figure 11 are examples of different types of needles 3 and stems 4 which can be coupled on the grip 1, with the suitable use of intermediate connectors 9A and 9B.

The bases 8A and 8B are positioned longitudinally surrounding a cylindrical element 17 of extension similar to the grip 1, which has on its median area two cylindrical projections 18 perpendicular and opposite among themselves, and on its frontal end 23 its diameter is slightly bigger.

The cylindrical element 17 slides inside a longitudinal and central hole 19 made on the said base 8B. The hole 19 has a circular section with two rectangular openings opposed by the center, the cylindrical element (17) passing along said hole 19, and having two guide projections 18 opposite to each other, with shape compatible to the said rectangular openings of the hole 19.

The activation of this mechanism, which takes place by pulling a handle 16, allows the bases 8A and 8B to be moved to their locking or working position.

Surrounding the cylindrical element 17 are provided two helical spring-type elastic devices 20A and 20B, positioned on the back part of the respective movable bases 8A and 8B; the spring 20A is trimmed by a rectangular element 26 with a circular hole, cross-positioned close to the grip center 1, and the spring 20B is trimmed by the back face 27. On the back end of the referred cylindrical element 17 the handle is coupled 16.

Close to the frontal part of the mono-block mechanism 7 are provided groups of cuts 28 on its sides, where a limiting element 29 of rectangular shape is fitted with a semi-circle shape opening and on the median area of the mono-block are provided groups of cuts 30, where a rectangular shape limiting element 31 is fitted with a semi-circle format opening. The limiting element 31 is one important feature of this type of device, since it allows the surgeon to control the needle perforation measure (1.2, 2.2 mm or more). The referred limiters 29 and 31 are coupled on the mono-block for adjustment of the distance to certain types of needle.

On the outer and lower part of the mono-block mechanism 7 is positioned the triggering and positioning system of the needle 3 and the stem 4, which is constituted of a rectangular section shaft 15 of greater length than the mono-block mechanism 7 and which has a trapezoid shape side cut 14A and a distance of a third from its back end and a "V" side cut 14B at a distance of a third of the frontal end.

In these cuts retention sliding "U"-shape elements 13A and 13B are positioned. This system allows a higher accuracy to the surgeon when inserting and positioning the needle 3 at the desired place for sample collection. The difference in the geometrical form of the referred openings 14A and 14B provides a difference of discharge time between the stem 4 and the needle 3 in milliseconds. This time difference is important for the success of the procedure, since it allows the needle 3 to leave from within the stem 4 only when it is necessary for the sample collection, eliminating the risk of contaminations by other tissues.

On the back end of the shaft 15 is positioned a safety lock 21 formed by a rectangular base, with a cylinder perpendicular to its face which goes internally through the distal portion of the part of the rectangular section 15, with a perpendicular projection on the other side forming an "L" 21, which is fitted into the traverse opening 22. When the lock is turned, the system is fully stopped, allowing the surgeon to go inside the patient's body without the risk of an accidental discharge of the needle at an unsuitable place, causing damage to the patient. But when the injury is located and the needle is necessary for collection, the surgeon has just to unlock the system and activate the frontal or back spare portion of the side longitudinal part 15, so as the system moves and the needle can, then, perform its task. Another great advantage offered by this moving system is that it enables the surgeon to operate the grip with only one of his hands. That requirement is extremely relevant, since usually this type of procedure is carried out with the aid of some other type of device which helps the surgeon to find the exact place of the sample collection, such as an ultra-sound device, for instance.

The triggering is performed in two phases; the first one carried out with the triggering of the stem, by pulling the handle up to its limit; and the second phase refers to the needle triggering, which is carried out by returning the handle to its rest position and rotating it 90 degrees to any direction and last by pulling it up to its limit again. The handle activation is carried out by elastic forces, and to trigger the device's discharge system should be applied a force higher than the spring forces which activate the set of needles.

On the conventional handles, the stem and the needle triggering system is simultaneous, which determined a high amount of force in its triggering. On the referred handle, the stem and needle triggering is performed in independent way, which renders the process easier by the simple fact that the force applied to the operation is lower.

## Claims

1. Sample collector for biopsy and similar, comprising a grip (1), a needle (3) fixed to the grip (1), a disposable stem (4) for collecting tissue sample, and an actuating mechanism (7) located inside said grip (1), **characterized in that** said actuating mechanism (7) comprises:
- one or more movable bases (8A, 8B) having on their upper faces a number of projections (10) and on their corners holes (12) inside which are located magnets (11), and
- interchangeable intermediate connectors (9A, 9B), which have through holes (34) matching said projections (10) for coupling with said bases (8A, 8B) by means of said magnets (11), and on their outer faces fitting elements (2A, 2B) for coupling with a standardized set of stems (4) and needles (3).

2. Sample collector as in claim 1, **characterized in that** said projections (10) are cylindrical, are linearly and longitudinally positioned on said bases (8A, 8B), and have sizes progressively larger.

3. Sample collector as in claims hereinbefore, **characterized in that** said base (8B) has a longitudinal and central hole (19) of circular section with two rectangular openings opposed by the center, a central cylindrical element (17) passing along said hole (19), and having two guide projections (18) opposite to each other, with shape compatible to the said openings of said hole (19).

4. Sample collector as in any claim hereinbefore, **characterized in that** close to the handle frontal part (1), is provided a group of cuts (28) on the mono-block sides where a first limiting element (29) can be fitted and close to the medium point of the mono-block is provided a group of cuts (30) on the sides where a second limiting element can be fitted (31), said limiting elements (29, 31) having a rectangular shape with an opening as a semi-circle.

5. Sample collector as in any claim hereinbefore, **characterized in that** said grip (1) comprises a quadrangular structure (5) coupled with an articulated lid (6), said lid (6) having magnets on its ends for locking with the quadrangular structure (5).
